(19) Europäisches Patentamt
European Patent Office
Office européen des brevets

(11) EP 2 166 088 A1

(12) **EUROPEAN PATENT APPLICATION**

(43) Date of publication:
**24.03.2010 Bulletin 2010/12**

(51) Int Cl.:
*C12N 9/04* (2006.01) *C12N 9/10* (2006.01)
*C12N 15/81* (2006.01)

(21) Application number: **08164826.3**

(22) Date of filing: **22.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **PomBio Tech GmbH**
**66123 Saarbrücken (DE)**

(72) Inventors:
- **Bureik, Matthias**
  **66117 Saarbrücken (DE)**
- **Buchheit, Daniela**
  **66121 Saarbrücken (DE)**
- **Dragan, Calin-Aurel**
  **66119 Saarbrücken (DE)**

(74) Representative: **Fleuchaus, Andrea et al**
**Fleuchaus & Gallo Partnerschaft**
**Patent- und Rechtsanwälte**
**Sollner Straße 36**
**81479 München (DE)**

(54) **Drug metabolism**

(57) The invention relates to the technical area of detoxification and metabolism of drugs as well as endogenous compounds. Particularly, the present invention relates to the physiological and pharmacological significance of glucuronidation and to the biotechnological production of glucuronides.

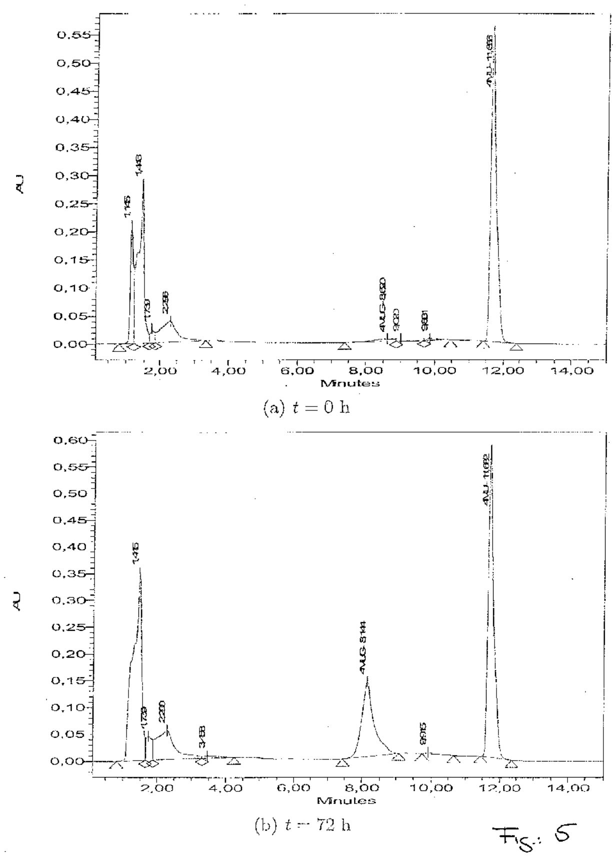


Fig. 5

EP 2 166 088 A1

Printed by Jouve, 75001 PARIS (FR)

SEQUENCE LISTING

<110> PomBioTech GmbH

<120> Drug Metabolism

<130> P11711EP00

<160> 2

<170> PatentIn version 3.3

<210> 1

<211> 1605

<212> DNA

<213> human UGT1A9

<400> 1

```
catatgatgg cttgcacagg gtggaccagc cccctcctc tatgtgtgtg tctgctgctg      60

acctgtggct ttgccgaggc agggaagcta ctggtagtgc ccatggatgg gagccactgg    120
ttcaccatga ggtcggtggt ggagaaactc attctcaggg ggcatgaggt ggttgtagtc    180
atgccagagg tgagttggca actgggaaga tcactgaatt gcacagtgaa gacttattca    240
acttcatata ccctggagga tctggaccgg gagttcaagg cttttgccca tgctcaatgg    300
aaagcacaag tacgaagtat atattctcta ttaatgggtt catacaatga catttttgac    360
ttatttttt caaattgcag gagtttgttt aaagacaaaa aattagtaga atacttaaag    420
gagagttctt ttgatgcagt gtttctcgat ccttttgata actgtggctt aattgttgcc    480
aaatatttct ccctcccctc cgtggtcttc gccaggggaa tactttgcca ctatcttgaa    540
gaaggtgcac agtgccctgc tcctctttcc tatgtcccca gaattctctt agggttctca    600
gatgccatga ctttcaagga gagagtacgg aaccacatca tgcacttgga ggaacattta    660
ttatgccacc gtttttcaa aaatgcccta gaaatagcct ctgaaattct ccaaacacct    720
gttacggagt atgatctcta cagccacaca tcaatttggt tgttgcgaac ggactttgtt    780
ttggactatc ccaaacccgt gatgcccaac atgatcttca ttggtggtat caactgccat    840
cagggaaagc cgttgcctat ggaatttgaa gcctacatta atgcttctgg agaacatgga    900
```

attgtggttt tctctttggg atcaatggtc tcagaaattc cagagaagaa agctatggca 960
attgctgatg ctttgggcaa aatccctcag acagtcctgt ggcggtacac tggaacccga 1020
ccatcgaatc ttgcgaacaa cacgatactt gttaagtggc taccccaaaa cgatctgctt 1080
ggtcacccga tgacccgtgc ctttatcacc catgctggtt cccatggtgt ttatgaaagc 1140
atatgcaatg gcgttcccat ggtgatgatg cccttgtttg gtgatcagat ggacaatgca 1200
aagcgcatgg agactaaggg agctggagtg accctgaatg ttctggaaat gacttctgaa 1260
gatttagaaa atgctctaaa agcagtcatc aatgacaaaa gttacaagga gaacatcatg 1320
cgcctctcca gccttcacaa ggaccgcccg gtggagccgc tggacctggc cgtgttctgg 1380
gtggagtttg tgatgaggca caagggcgcg ccacacctgc gccccgcagc ccacgacctc 1440
acctggtacc agtaccattc cttggacgtg attggtttcc tcttggccgt cgtgctgaca 1500
gtggccttca tcacctttaa atgttgtgct tatggctacc ggaaatgctt ggggaaaaaa 1560
gggcgagtta agaaagccca caaatccaag acccattgag gatcc 1605

<210> 2

<211> 1497

<212> DNA

<213> human UGDH

<400> 2

catatgatgt ttgaaattaa gaagatctgt tgcatcggtg caggctatgt tggaggaccc 60
acatgtagtg tcattgctca tatgtgtcct gaaatcaggg taacggttgt tgatgtcaat 120
gaatcaagaa tcaatgcgtg gaattctcct acacttccta tttatgagcc aggactaaaa 180
gaagtggtag aatcctgtcg aggaaaaaat cttttttttt ctaccaatat tgatgatgcc 240
atcaaagaag ctgatcttgt atttatttct gtgaatactc caacaaaaac ctatggaatg 300
gggaaaggcc gggcagcaga tctgaagtat attgaagctt gtgctagacg cattgtgcaa 360
aactcaaatg ggtacaaaat tgtgactgag aaaagcacag ttccagtgcg ggcagcagaa 420
agtatccgtc gcatatttga tgcaaacaca aaacccaact tgaatttaca ggtgctgtcc 480
aaccctgagt ttctggcaga gggaacagcc atcaaggacc taaagaaccc agacagagta 540
ctgattggag gggatgaaac tccagagggc cagagagctg tgcaggccct gtgtgctgta 600
tatgagcact gggttcccag agaaaagatc ctcaccacta atacttggtc ttcagagctt 660
tccaaactgg cagcaaatgc ttttcttgcc cagagaataa gcagcattaa ctccataagt 720
gctctgtgtg aagcaacagg agctgatgta gaagaggtag caacagcgat tggaatggac 780
cagagaattg gaaacaagtt tctaaaagcc agtgttgggt ttggtgggag ctgtttccaa 840
aaggatgttc tgaatttggt ttatctctgt gaggctctga atttgccaga agtagctcgt 900
tattggcagc aggtcataga catgaatgac taccagagga ggaggtttgc ttcccggatc 960
atagatagtc tgtttaatac agtaactgat aagaagatag ctattttggg atttgcattc 1020
aaaaaggaca ctggtgatac aagagaatct tctagtatat atattagcaa atatttgatg 1080
gatgaaggtg cacatctaca tatatatgat ccaaaagtac ctagggaaca aatagttgtg 1140

```
gatctttctc atccaggtgt ttcagaggat gaccaagtgt cccggctcgt gaccatttcc   1200
aaggatccat atgaagcatg tgatggtgcc catgctgttg ttatttgcac tgagtgggac   1260
atgtttaagg aattggatta tgaacgcatt cataaaaaaa tgctaaagcc agcctttatc   1320
ttcgatggac ggcgtgtcct ggatgggctc cacaatgaac tacaaaccat tggcttccag   1380
attgaaacaa ttggcaaaaa ggtgtcttca aagagaattc catatgctcc ttctggtgaa   1440
attccgaagt ttagtcttca agatccacct aacaagaaac ctaaagtgta gggatcc      1497
```

SEQUENCE LISTING

<110> PomBioTech GmbH

<120> Drug Metabolism

<130> P11711EP00

<160> 2

<170> PatentIn version 3.3

<210> 1
<211> 1605
<212> DNA
<213> human UGT1A9

<400> 1
catatgatgg cttgcacagg gtggaccagc cccttcctc tatgtgtgtg tctgctgctg 60

acctgtggct ttgccgaggc agggaagcta ctggtagtgc ccatggatgg gagccactgg 120

ttcaccatga ggtcggtggt ggagaaactc attctcaggg ggcatgaggt ggttgtagtc 180

atgccagagg tgagttggca actgggaaga tcactgaatt gcacagtgaa gacttattca 240

acttcatata ccctggagga tctggaccgg gagttcaagg cttttgccca tgctcaatgg 300

aaagcacaag tacgaagtat atattctcta ttaatgggtt catacaatga catttttgac 360

ttattttttt caaattgcag gagtttgttt aaagacaaaa aattagtaga atacttaaag 420

gagagttctt ttgatgcagt gtttctcgat ccttttgata actgtggctt aattgttgcc 480

aaatatttct ccctcccctc cgtggtcttc gccaggggaa tactttgcca ctatcttgaa 540

gaaggtgcac agtgccctgc tcctctttcc tatgtcccca gaattctctt agggttctca 600

gatgccatga ctttcaagga gagagtacgg aaccacatca tgcacttgga ggaacattta 660

ttatgccacc gtttttcaa aaatgcccta gaaatagcct ctgaaattct ccaaacacct 720

gttacggagt atgatctcta cagccacaca tcaatttggt tgttgcgaac ggactttgtt 780

ttggactatc ccaaacccgt gatgcccaac atgatcttca ttggtggtat caactgccat 840

cagggaaagc cgttgcctat ggaatttgaa gcctacatta atgcttctgg agaacatgga 900

attgtggttt tctctttggg atcaatggtc tcagaaattc cagagaagaa agctatggca 960

attgctgatg ctttgggcaa aatccctcag acagtcctgt ggcggtacac tggaacccga 1020

ccatcgaatc ttgcgaacaa cacgatactt gttaagtggc taccccaaaa cgatctgctt 1080

ggtcacccga tgacccgtgc ctttatcacc catgctggtt cccatggtgt ttatgaaagc 1140

atatgcaatg gcgttcccat ggtgatgatg cccttgtttg gtgatcagat ggacaatgca 1200

aagcgcatgg agactaaggg agctggagtg accctgaatg ttctggaaat gacttctgaa 1260

gatttagaaa atgctctaaa agcagtcatc aatgacaaaa gttacaagga gaacatcatg 1320

```
cgcctctcca gccttcacaa ggaccgcccg gtggagccgc tggacctggc cgtgttctgg   1380
gtggagtttg tgatgaggca caagggcgcg ccacacctgc gccccgcagc ccacgacctc   1440
acctggtacc agtaccattc cttggacgtg attggtttcc tcttggccgt cgtgctgaca   1500
gtggccttca tcacctttaa atgttgtgct tatggctacc ggaaatgctt ggggaaaaaa   1560
gggcgagtta agaaagccca caaatccaag acccattgag gatcc                   1605
```

<210> 2
<211> 1497
<212> DNA
<213> human UGDH

<400> 2

```
catatgatgt ttgaaattaa gaagatctgt tgcatcggtg caggctatgt tggaggaccc     60
acatgtagtg tcattgctca tatgtgtcct gaaatcaggg taacggttgt tgatgtcaat    120
gaatcaagaa tcaatgcgtg gaattctcct acacttccta tttatgagcc aggactaaaa    180
gaagtggtag aatcctgtcg aggaaaaaat ctttttttt ctaccaatat tgatgatgcc     240
atcaaagaag ctgatcttgt atttatttct gtgaatactc caacaaaaac ctatggaatg    300
gggaaaggcc gggcagcaga tctgaagtat attgaagctt gtgctagacg cattgtgcaa    360
aactcaaatg ggtacaaaat tgtgactgag aaaagcacag ttccagtgcg ggcagcagaa    420
agtatccgtc gcatatttga tgcaaacaca aaacccaact tgaatttaca ggtgctgtcc    480
aaccctgagt ttctggcaga gggaacagcc atcaaggacc taaagaaccc agacagagta    540
ctgattggag gggatgaaac tccagagggc cagagagctg tgcaggccct gtgtgctgta    600
tatgagcact gggttcccag agaaaagatc ctcaccacta atacttggtc ttcagagctt    660
tccaaactgg cagcaaatgc ttttcttgcc cagagaataa gcagcattaa ctccataagt    720
gctctgtgtg aagcaacagg agctgatgta gaagaggtag caacagcgat tggaatggac    780
cagagaattg gaaacaagtt tctaaaagcc agtgttgggt ttggtgggag ctgtttccaa    840
aaggatgttc tgaatttggt ttatctctgt gaggctctga atttgccaga agtagctcgt    900
tattggcagc aggtcataga catgaatgac taccagagga ggaggtttgc ttcccggatc    960
atagatagtc tgtttaatac agtaactgat aagaagatag ctattttggg atttgcattc   1020
aaaaaggaca ctggtgatac aagagaatct tctagtatat atattagcaa atatttgatg   1080
gatgaaggtg cacatctaca tatatatgat ccaaaagtac ctagggaaca aatagttgtg   1140
gatctttctc atccaggtgt ttcagaggat gaccaagtgt cccggctcgt gaccatttcc   1200
aaggatccat atgaagcatg tgatggtgcc catgctgttg ttatttgcac tgagtgggac   1260
atgtttaagg aattggatta tgaacgcatt cataaaaaaa tgctaaagcc agcctttatc   1320
```

```
ttcgatggac ggcgtgtcct ggatgggctc cacaatgaac tacaaaccat tggcttccag   1380
attgaaacaa ttggcaaaaa ggtgtcttca aagagaattc catatgctcc ttctggtgaa   1440
attccgaagt ttagtcttca agatccacct aacaagaaac ctaaagtgta gggatcc      1497
```

## Description

The invention relates to the technical area of detoxification and metabolism of drugs as well as endogenous compounds. Particularly, the present invention relates to the physiological and pharmacological significance of glucuronidation and to the biotechnological production of glucuronides.

Among all glycosylation reactions, glucuronidation is the major detoxification process, especially in humans where glucuronide conjugates constitute most of the detoxification products excreted in bile and urine. In addition to its beneficial detoxification properties, glucuronidation is also known to modulate toxicities associated with exposure to several carcinogens. Glucuronidation is a chemical reaction that is performed by the enzymes of the family of UDP-glucuronosyltranferases (UTGs). Glucuronidation results in the covalent binding of activated glucuronic acid (GlcUA) to substrates, which are thereby transformed into polar glucuronides. The activated GlcUA is UDP-glucuronate (UDP-GlcUA), the product of a reaction catalyzed by UDP-glucose dehydrogenase (UGDH).

The glucuronidation, or more generally spoken the conjunction of small hydrophobic molecules with a sugar moiety, is an evolutionary conserved reaction occurring in a wide range of prokaryotic and eukaryotic organisms (for review see Radominska-Pandya et al., Current Drug Metabolism 6 (2005) 141-160).

In yeast, no endogenous glucuronidation machinery has been described, and in the genome sequences of yeast species that are known so far no homologues of UGTs have been found. Still, it has been reported by Ikushiro et al. (Biochimica et Biophysica Acta 1672 (2004) 86-92) that they were able to modify Saccharomyces cerevisiae AH22 to express rat UTG1A6. However, the enzyme was only functional active in yeast microsome preparations, which were prepared as described in Oeda et al. (DNA 4 (1985) 203-210). The stability and standardisation of such yeast microsome preparations is difficult and reliability is doubtful. Accordingly, the expression system presented by Ikushiro et al. has only limited value for quantitative studies of metabolism of drugs or endogenous substances. No glucuronidations by whole-cell biotransformation using recombinantly UGT expressing yeasts have been described so far. In summary, there is still a need for functional UTG expression systems, which allow - with reasonable effort - quantitative studies of the metabolism and in particular studies on the glucuronidation of drugs, xenobiotics or endogenous compounds as well as the efficient production af the respective glucuronides.

The present invention for the first time provides a whole-cell biotransformation system for the production of glucuronides, characterized by a functional coexpression of an UTG isoenzyme together with UGDH. Although the state of the art indicated difficulties with yeast based expression systems for UTG, the inventors of the present invention were able to establish a functional coexpression system for an UTG and an UGDH in yeast, which - as a particularly interesting feature - for the first time allows the production of glucuronides by living yeast cells and the release of the metabolites of the functional UTG into the supernatant. Accordingly, this expression system can be easily and without the effort of intermediate preparation steps (such as e.g. microsomal preparation) be used for various test assays or for the production of UTG metabolites, such as various glucuronides.

For establishing the expression system for functional UTGs the present invention provides recombinant fission yeast. The fission yeast is selected from the group comprising *Schizosaccharomycetes,* such as *Schizosaccharomyces pombe* as well as strains derived from *Schizosaccharomyces pombe* and related yeast strains.

Lindner has scientifically described *Schizosaccharomyces pombe* in 1893. Its genomic sequence was published in 2002 (Wood et al. (2002) Nature, 415, 871-880). Interestingly, *Schizosaccharomyces pombe,* although being a yeast, from an evolutionary point of view is very distinct from the well-known bakers yeast *Saccharomyces cerevisiae.*

The term "related fission yeast strains" defines herein yeast strains, which have the same functional characteristics as *Schizosaccharomyces pombe.* Furthermore, strains derived from *Schizosaccharomyces pombe* refer to recombinant as well as conventionally or molecularly modified strain of this yeast, whereby the modifications are insertions, exchanges, additions or deletions of genomic sequence, which may show in the phenotype.

The fission yeast according to the invention has been transformed to express the nucleic acid sequence encoding UDP-glucuronosyltransferase (UTG), UDP-glucose dehydrogenase (UGDH) and/or functional variants thereof.

In the context of the present invention the following definitions shall be relevant.

The term "nucleic acid sequence" as used herein refers to an oligonucleotide, nucleotide, or polynucleotide, and fragments or portions thereof, and to DNA, cDNA or RNA of genomic or synthetic origin, which may be single- or double-stranded, and represent the sense or antisense strand.

"UDP-glucuronosyltransferase" or "UTG", as used herein, refers to the amino acid sequences of substantially purified UTG polypeptide obtained from any microbial, plant or animal species, particularly mammalian, including bovine, ovine, porcine, murine, equine, simian, and preferably human, from any source whether natural, synthetic, semi-synthetic or recombinant. Particularly, the term includes the previously identified and described human UTGs, such as UGT1A1, UGT1A3, IJGT1A4, UGT1A5, UGT1A6, UGT1A7, UGT1A8, UGT1A9, UGT1A10, UGT2B4, UGT2B7, UGT2B10, UGT2B11, UGT2B15, UGT2B17, UGT2B28, UGT2A1, and UGT2A2 (for a review see Trubetskoy et al. (2008), J Pharm Pharmacol, 60, 1061-1067).

UGTs are a family of type I membrane proteins located in the ER (Meech and Mackenzie, 1998, Arch Biochem Biophys., 356, 77-85) and the outer and inner nuclear membranes (Radominska-Pandya et al., 2002, Arch Biochem Biophys., 399, 37-48). The activity of UGTs is totally dependent on the presence of phospholipids (cf. Radominska-Pandya et al., 2005, Curr Drug Metab., 6, 141-160)). The genes encoding UGTs have been assigned to two families based on sequence similarities. The isoforms of the UGT1A family are all derived from a single gene by alternative splicing of four constant exons (exons 2 to 5) to a variable exon 1. The UGT2 family, in contrast, is encoded by separate genes and is divided into the UGT2A and UGT2B subfamilies (Mackenzie et al., 2005, Pharmacogenet Genomics., 15, 677-685).

"UDP-glucose dehydrogenase" or "UGDH" (EC 1.1.1.22), as used herein, refers to the amino acid sequences of substantially purified UGDH polypeptide obtained from any microbial, plant or animal species, particularly mammalian, including bovine, ovine, porcine, murine, equine, simian, and preferably human, from any source whether natural, synthetic, semi-synthetic or recombinant. Particularly, the term includes the previously identified and described UGDHs, such as human or bovine UGDH.

UGDH catalyzes an $NAD^+$-dependent two-fold oxidation of UDP-glucose to generate UDP-glucuronate (UDP-GlcUA). UDP-glucuronate is the required substrate for glucuronidation of many substances, including xenobiotics, opioids, androgens, and heme proteins through the action of UDP-glucuronosyl transferases in the liver (cf. Huh et al. (2004), J Biol Chem., 279, 37491-37498).

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

A "variant", as used herein, refers either to a nucleic or amino acid sequence that is altered by one or more nucleotides or amino acids. The term "variant" particularly includes the nucleic acid sequences, which have been optimized for strain or organism specific codon usage. Additionally, the term encloses nucleic acid sequences, which have nucleotide exchanges due the degeneration of the genetic code or more interestingly due to individual polymorphisms.

Further, the "variant" of an amino acid sequence may have conservative amino acid changes, wherein a substituted amino acid has similar structural or chemical properties, e.g., replacement of leucine with isoleucine. More rarely, a variant may have non-conservative changes, e.g., replacement of a glycine with a tryptophan. Similar minor variations may also include amino acid deletions or insertions, or both. Guidance in determining which amino acid residues may be substituted, inserted, or deleted without abolishing biological or immunological activity may be found using computer programs well known in the art.

Further, the term "functional variant" is understood herein as a variant amino acid sequence, a peptide derived from the original amino acid sequence or a functional domain of the original amino acid sequence, which still shows the same enzymatic, biological or immunological activity as the original protein, peptide or active domain. A variant is still considered a "functional variant" if the amount of activity varies.

The term "transformed", as defined herein, describes a process (transformation) by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but are not limited to, viral infection, electroporation, lipofection, and particle bombardment. Such "transformed" cells include stably transformed cells in which the inserted DNA is capable of replication either as an autonomously replicating plasmid or as part of the host chromosome. They also include cells, which transiently express the inserted DNA or RNA for limited periods of time.

The fission yeast-based expression system according to the present invention is capable of expressing functional UTG and/or functional UGDH. To this end, a fission yeast strain, preferably *S. pombe,* is transformed with either integrative or autosomally replicating vectors, which carry under the regulatory control of at least one promoter element the nucleic acid sequence encoding UTG and/or UGDH.

According to one embodiment the promoter elements used in the present invention are inducible promoter elements, which can be induced, switched on and off according to the needs of the experiment and the expression system.

It has been found - as a first sign of functional expression of UTG and UGDH - that the phenotypic morphology of the transformed yeast cells is affected. Expression studies showed that some populations of recombinant yeast cells show deviations from the usual rod-like shape and also show unequal cell division. Furthermore, it was found that a functional expression of UTG and UGDH can cause some growth retardation.

To overcome any potential disadvantages such as e.g. growth retardation, according to one further embodiment the expression levels of UTG and UGDH, respectively, have been adjusted. Such adjustment can be e.g. caused by promoter elements of different strength in expressing the relevant nucleic acid sequence. Alternatively, adjustment can be reached by transforming the parent strain with more than one copy of the nucleic acid of interest. Other methods to adjust the expression level are well known in the art.

This allows for the first time to establish a method for detecting and synthesizing UTG-dependent metabolites of drugs, xenobiotics or selected endogenous compounds in the supernatant of a microbial cell culture. Former systems for the whole-cell production of glucuronides were restricted to mammalian or insect cell systems (Trubetskoy et al. (2008), J Pharm Pharmacol, 60, 1061-1067), which in comparison to a yeast system are very disadvantegous as they are comparably inconvenient, expensive and time-consuming.

For the method presented in this invention, the expression system for functional UTG, namely a population of the recombinant *Schizosaccharomyces pombe,* which is transformed with the nucleic acid sequence encoding an UTG isoenzyme and the nucleic acid sequence encoding an UGDH is cultivated under suitable conditions. Such suitable conditions are the standard cell culture conditions for fission yeast. Briefly, cultivation is done in EMM medium, supplemented with thiamine and other necessary or essential supplements known to the practitioner. Optionally, and in case of inducible expression of the UTG and UGDH this expression is induced with a suitable inducer. Then, the test drug is added to the fermentation broth. For bioconversion of the test drug the cell culture is further incubated for 6 to 72 hours at suitable conditions, e.g. 30 °C. At the end of the bioconversion period the supernatant is collected, optionally centrifuged for clarification, and prepared for HPLC analysis. The HPLC analysis revealed the nature of the metabolites of the test drug, which were generated by the functional UTG. Additionally, mass spectroscopy confirmed the HPLC results.

It is to note that the method of the invention for the first time allows detecting the UTG dependent metabolites in a whole-cell based assay system using a recombinant microbial host. This system can be established for any desired UTG. Of main interest are the various human UTGs, which mainly belong to 3 families (UTG1A; UTG2A; UTG2B) and which include many variants. By learning and knowing about the potentially different metabolites of a certain drug clinicians and researchers might understand the effect and side effects of a drug much better. On the long run, this will lead to more individualized and thus better therapeutic strategies.

Furthermore, also the functional expression according the invention of UTGs of non-human, but other mammalian origin is interesting and also important. Particularly, for animals, which might be used in preclinical studies it is important to know, whether they would metabolize a certain drug in the same or a different way compared to humans. The invention provides a method, which avoids the use of - labour intensive - liver cell extractions to learn about such potential differences in metabolites.

The present method also allows to scale up the bioconversion process. Under adjusted culturing conditions not only the cell culture and fermentation process can be increased but also the bioconversion period can be enlarged. Such a large-scale fermentation provides increased amounts of the UTG dependent metabolite(s) of the test drug. These metabolites can than be isolated and purified from the supernatant.

According to a further embodiment the present system is particularly useful to synthesise, extracting and purify UDP-glucuronic acid (UDP-GA). For this purpose, fission yeast strains are employed that solely express a nucleic acid sequence encoding UGDH.

According to still a further embodiment the present expression system, comprising the S. *pombe* transformed with the nucleic acid sequence encoding UTG and/or the nucleic acid sequence encoding UGDH can also be used for the manufacturing of a enzyme preparation useful in standardised glucuronidation assays.

According to still another embodiment the expression system for functional UTG is used to identify substances, molecules or peptides, which either modify the UTG activity or block the UTG activity.

For this method the bioconversion of a selected test drug (such as 4MU) is used as standardising assay, which means the metabolites of this test drug are analysed. Then, potential substances of interest are added to the bioconversion assay in order to test and identify UTG modulating activity. Any changes in the amount of the produced metabolite or any changes in the distribution of produced metabolite are analysed and evaluate. This method allows detecting potential inhibitors or modulators of the various UTGs, which might become useful as therapeutic targets.

## Short description of the Figures

**Figure 1: Vector map of pCAD1.** Pnmt1 = nmt1 promoter; Insert = gene of interest (in this figure 1.5 kb); ura4 = selection marker; Leu1Loc = gene fragments of the *leu1* gene; AmpR = ampicillin resistance gene. The position of selected restriction sites is indicated.

**Figure 2: Vector map of pREP1.** Pnmt1 = nmt1 promoter; Tnmtl = nmt1 terminator; LEU2 = selection marker; ars1 = autonomously replicating sequence. The position of selected restriction sites is indicated.

**Figure 3: The cDNA sequence of human UGT1A9 used in this project.** The amino acid sequence that corresponds to this cDNA is identical to that of the human UGT1A9 enzyme. Restriction sites for *Nde* I (5'-end) and *Bam*H I (3'-end), resp., are shown in bold.

**Figure 4: The cDNA sequence of human UGDH used in this project.** The amino acid sequence that corresponds to this cDNA is identical to that of the human UGDH enzyme. Restriction sites for *Nde* I (5'-end) and *Bam*H I (3'-end), resp., are shown in bold.

**Figure 5: Exemplary HPLC chromatogram at λ = 320 nm** gained from the supernatant sample of a CAD203 culture incubated with 500 M 4-methylumbelliferone. The biomass was raised and treated as described in described in Example 2. Analysis was done as described in Example 3. Chromatogram (a) stems from a sample drawn at *t* = 0 h while (b) was taken at *t* = 72 h. The labeling of peaks as 4MU (4-methylumbelliferone) and 4MUG (4-methlyumbelliferone glucuronide) was done by the peak detection algorithm on the basis of retention times.

**Figure 6: Exemplary HPLC chromatogram at λ = 320 nm** gained from the supernatant sample of a NCYC2036 culture incubated with 500 M 4-methylumbelliferone (negative control). The biomass was raised and treated as described in Example 2. Analysis was done as described in Example 3. Chromatogram (a) stems from a sample drawn at *t* = 0 h while (b) was taken at *t* = 72 h. Labeling of peaks was done as in Figure 5. Note the absence of 4MUG.

**Figure 7: Extracted UV/VIS peak spectra from the PDA channel** that was used to extract the chromatogram shown in Figure 5 stemming from a CAD203 biotransformation of 4MU. The peak spectra are ordered according to ascending retention times and are shown above the chromatographic section used for peak extraction. Subfigure (a) stems from a sample drawn at *t* = 0 h while (b) was taken at *t* = 72 h. The labeling of peaks as 4MU (4-methylumbelliferone) and 4MUG (4-methlyumbelliferone glucuronide) was done by the peak detection algorithm on the basis of retention times. Spectrum search was done against the library stored 4MU and 4MUG spectra which in case of a match were additionally drawn in the graph belonging to a particular peak.

**Figure 8: Extracted UV/VIS peak spectra from the PDA channel** that was used to extract the chromatogram shown in Figure 6 stemming from a NCYC2036 biotransformation of 4MU (negative control). The peak spectra are ordered according to ascending retention times and are shown above the chromatographic section used for peak extraction. Subfigure (a) stems from a sample drawn at *t* = 0 h while (b) was taken at *t* = 72 h. Labeling of peaks was done as in Figure 7. Note the absence of 4MUG.

## Examples

### 1. Expression system for functional UDP-glucuronosyltransferase - UTG 1A9

For establishing a biotransformation system for the generation and production of glucuronides and glucuronide metabolites of drugs or xenobiotics the fission yeast *Schizosaccharomyces pombe* was transformed according to the following work protocol to express the human UDP-glucuronosyltransferase UGT1A9 and coexpress the human UDP-glucose dehydrogenase (UGDH).

#### *1.1 Gene design and cDNA synthesis*

The cDNA sequences for human UGT1A9 and human UGDH were synthesized by Entelechon GmbH (Regensburg, Germany) and provided as cloned fragments in suitable *Escherichia coli* cloning vectors.

The amino acid sequences of human UGT1A9 and of human UGDH, resp., were retrieved from UniProtKB/Swiss-Prot (ugt1a9: *http://www.expasy.org/uniprot/O60656;*
ug6dh: *http://www.expasy.org/uniprot/O60701*), the corresponding cDNA sequences were then optimized for strong expression in fission yeast (Fig. 3 & 4) and synthesized by Entelechon GmbH (Regensburg, Germany) with 5' terminal *Nde* I and 3' terminal *Bam*H I restriction sites, resp., to allow direct cloning into the fission yeast expression vectors pCAD1 and pREP1.

#### *1.2 Expression vectors*

The fission yeast expression vector pCAD1 (Fig. 1) has been described (Dragan *et al.* 2005 *FEMS Yeast Res.* **5**, 621-625). This vector stably integrates into the *leu1* gene of chromosome II of fission yeast and thereby deletes the target gene. pCAD1 allows strong gene expression under the control of the *nmt1* promoter (no message in *t*hiamine), which is regulated by the presence of thiamine in the growth medium. The vector contains fragments of the *leu1* gene that serve as integration target sequences, a gene for -lactamase (AmpR) and an ura4 gene for the orotidine monophosphate decarboxylase, which complements the gene defect *ura4.dl18* in fission yeast and therefore can be used for positive selection. A *Not*I restriction digest of pCAD1 yields an integration construct that contains the expression cassette, the *ura4* marker, and flanking *leu1* sequences and a second fragment that contains the sequences needed for propagation in *E. coli* (which thus dispensable for the fission yeast transformation). The size of the integration fragment depends on the length of the gene of interest (insert) in pCAD1.
The second fission yeast expression vector used in this project, pREP1 (Fig. 2), also allows protein expression under control of the *nmt1* promoter (Maundrell 1993 *Gene* **123**, 127-130). Unlike pCAD1, the pREP1 vector contains the *LEU2* gene from bakers yeast *Saccharomyces cerevisiae* as a selection marker, which complements *leu1* gene defects in fission yeast. Also, it does not integrate into the fission yeast genome but replicates autonomously.

### *1.3 DNA restriction*

General DNA manipulation methods were performed using standard techniques (Sambrook et al. 1989 Molecular Cloning: A Laboratory Manual. 2nd Edn. Cold Spring Harbor Press, Cold Spring Harbor, NY). The excision of nucleic acid fragments out of plasmids was carried out using restriction endonucleases. Restriction enzymes *Bam*H I, *Nde* I and *Not* I were used according to the instructions of the manufacturer (Promega; Madison, WI, USA). Briefly, the reaction mixture consisted of the following components:

| | | |
|---|---|---|
| DNA | approx. 5 μg | (for preparative restrictions) |
| | approx.100 ng | (for analytical restrictions) |
| enzyme buffer | 3 μL | |
| enzyme | 0.5 μL | |
| bovine serum albumin | 1 μL | |
| deionized water | *ad* 30 μL | |

### *1.4 DNA gel electrophoresis*

Nucleic acid fragments were separated and analysed by gel electrophoresis using 1% (w/v) agarose containing gels. The running buffer was 0.5X TBE with the following composition:

| | |
|---|---|
| Tris base | 45 mM |
| $H_3BO_3$ | 45 mM |
| EDTA pH 8.0 | 1 mM |

DNA fragments were visualised at 312 nm wavelength by addition of 0.02% ethidium bromide (w/v) using a UST-20M-8K transilluminator (Biostep; Jahnsdorf, Germany).

### *1.5 Isolation and precipitation of DNA fragments out of agarose gels*

After separation of the DNA by gel electrophoresis, the nucleic acid fragments were centrifuged at 7000 *g* (room temperature, 2 min) in the presence of a silanized glass wool matrix (Supelco; Bellefonte, PA, USA) followed by a precipitation of the DNA at -20°C for 30 min using a solution consisting of 0.1 volume parts 3 M sodium acetate and 2 volume parts ethanol. Afterwards, the precipitated DNA was centrifuged at 20.000 *g* (4°C, 30 min).

### *1.6 Ligation of nucleic acids*

The ligation of the insert (nucleic acid fragments of interest) and vector was done using T4 ligase according to the instructions of the manufacturer (Fermentas; St. Leon-Rot, Germany). Briefly, sample composition was as follows:

| | |
|---|---|
| DNA (vector) | approx. 100 ng |
| DNA (insert) | approx. 300 ng |
| T4 ligase | 3 μL |
| T4 buffer | 2 μL |
| deionized water | *ad* 20 μL |

***1.7 Microbial media***

*1.7.1 Media for* Escherichia coli

• SOC medium

| | |
|---|---|
| tryptone | 10 g |
| yeast extract | 5 g |
| KCl | 186 mg |
| $MgCl_2 \cdot 6 H_2O$ | 2.03 g |
| NaCl | 580 mg |
| glucose | 3.6 g |
| deionized water | *ad* 1L |

• NB medium

| | |
|---|---|
| peptone from casein | 7 g |
| peptone from meat | 5 g |
| peptone from gelatine | 5 g |
| yeast extract | 3 g |
| NaCl | 5 g |
| deionized water | *ad* 1 L |
| pH (adjust if necessary) | 7.2 |

*1,7.2 Media for* Schizosaccharomyces pombe

• EMM (Edinburgh Minimal Medium)

| | |
|---|---|
| potassium phthalate | 3 g |
| $Na_2HPO_4 \cdot 2 H_2O$ | 2.2 g |
| $NH_4Cl$ | 5 g |
| glucose | 20 g |
| pH (adjust if necessary) | 5.4 to 5.8 |

| | |
|---|---|
| salt stock solution | 20 mL |
| vitamin stock solution | 1 mL |
| mineral stock solution | 100 μL |
| deionized water | *ad* 1 L |

• salt stock solution

| | |
|---|---|
| $MgCl_2 \cdot 6 H_2O$ | 52.5 g |
| $CaCl_2 \cdot 6 H_2O$ | 1 g |
| KCl | 50 g |
| $Na_2SO_4$ | 2 g |
| deionized water | *ad* 1 L |

• vitamin stock solution

| | |
|---|---|
| sodium pantothenate | 1 g |
| nicotinic acid | 10 g |

(continued)

| | |
|---|---|
| nositol | 10 g |
| biotin | 10 mg |
| deionized water | *ad* 1 L |

• mineral stock solution

| | |
|---|---|
| $H_3BO_4$ | 5 g |
| $MnSO_4$ | 4 g |
| $ZnSO_4 \cdot 7H_2O$ | 4 g |
| $FeCl_3 \cdot 6\ H_2O$ | 2 g |
| molybdic acid | 1.6 g |
| KI | 1 g |
| $CuSO_4 \cdot 5\ H_2O$ | 400 mg |
| citric acid | 10 g |
| deionized water | *ad* 1 L |

*1.8 Preparation of competent* E. coli *cells*

5 μL of a permanent culture of TOP10F' cells (Invitrogen; Breda, Netherlands) were streaked on a NB dish. A 3 mL preculture was cultivated in NB medium containing 10 mM $Mg^{2+}$ at 37°C and 150 rpm. After 12 h cells were transferred into 50 mL NB medium (main culture) supplemented with 10 mM $Mg^{2+}$. Cells were cultivated as before until they reached an optical density at 600 nm ($OD_{600}$) between 0.3 and 0.5. After centrifugation (5 min, 4°C, 4000 *g*) the cell pellet was resuspended in 14 mL TFP I buffer and incubated on ice for 1 h. Afterwards cells were centrifuged as before and resuspended in 2 mL TFP II buffer. Aliquots of 100 μL were shock frosted in liquid nitrogen and stored at -80°C untill usage.

• TFP I

| | |
|---|---|
| potassium acetate | 30 mM |
| KCl | 100 mM |
| $CaCl_2$ | 10 mM |
| glycerol | 12% (v/v) |

After filter sterilisation add 5 mL of a 1 M $MnCl_2$ solution.

• TFP II

| | |
|---|---|
| MOPS | 10 mM |
| KCl | 10 mM |
| $CaCl_2$ | 75 mM |
| glycerol | 12% (v/v) |
| deionized water | *ad* 20 mL |
| pH (adjust if necessary) | 6.5 to 7.0 |

***1.9 Transformation of* Escherichia coli**

The gram-negative bacterium *Escherichia coli* DH5α (genotype: *F deoR recA1 endA1 hsdR17(rk⁻, mk⁺) supE44* $\lambda^-$ *thi-1 gyrA96 relA1)* was used for plasmid amplification. Transformation of *E. coli* cells was performed by the heat shock method (42°C for 30 s). All samples contained 5 μL of a ligation mixture (see 2.6). After the heat shock cells were incubated in SOC medium at 37°C and 150 rpm for 1 h, followed by an overnight incubation in NB medium supplemented with ampicillin (1 g/L) at 37°C.

### *1.10 Plasmid isolation*

After receipt of the cDNAs cloned into the vector pCR4-TOPO (Invitrogen, Karlsruhe, Germany), competent *E. coli* cells were transformed with the corresponding plasmids and cultivated followed by a plasmid preparation. Plasmids were isolated using the NucleoBond® PC100 kit (Macherey & Nagel; Düren, Germany).

### *1.11 Cloning of fission yeast expression plasmids*

The UGTIA9 cDNA was excised from pCR4-TOPO with *Nde* I/*Bam*H I and ligated with *Nde* I/*Bam*H I restricted fission yeast expression vectors pCAD1 and pREP1 to yield the new expression plasmids pCAD1-UGT1A9 and pREP1-UGT1A9, resp.. Similarly, the UGDH cDNA was excised from pCR4-TOPO with *Nde* I/*Bam*H I and ligated with *Nde* I/*Bam*H I restricted fission yeast expression vectors pCAD1 and pREP1 to yield the new expression plasmids pCAD1-UDGH and pREP-UDGH, resp..

### *1.12 Validation of the cloning products*

After preparation of the new fission yeast expression plasmids their identity was validated by analytical restriction with *Nde* I, *Bam*H I and *Not* I. Additionally, the correctness of all expression constructs was confirmed by automatic sequencing.

### *1.13 Transformation of fission yeast*

Media and genetic methods for studying fission yeast have been described in detail (Moreno et al. 1991 Methods Enzymol 194, 795-823; Alfa et al. 1993 Experiments with fission yeast. A laboratory course manual. Cold Spring Harbor Press, Cold Spring Harbor, NY). Transformation of fission yeast strains were done as described (Dragan et al. 2005 FEMS Yeast Res 5, 621-625). Briefly, fission yeast transformation was done by the lithium acetate method combined with a heat shock at 42°C for 5 min. About 1 to 5 $\mu$g of DNA were used per transformation reaction. After transformation cells were incubated on EMM (Edinburgh Minimal Medium) dishes with 0.1 % of the required auxotrophy marker solutions and 5 $\mu$M of thiamine at 30°C until colonies were visible.

For the construction of single expressor strains, pCAD1-UGT1A9 and pCAD1-UGDH were restricted with *Not* I and separated from the bacterial vector backbone by agarose gel electrophoresis. The precipitated *Not* I integration fragments were dissolved in water and used directly to transform fission yeast strain NCYC2036 (Losson & Lacroute 1983 Cell 32, 371-377; genotypes of all strains are listed in Table 1). After transformation cells were plated on EMM + leucine + thiamine dishes and grown for three days until colonies appeared. Correct integration of the expression constructs into the *leu1* gene was confirmed by replica plating of clones onto media plates without leucine. Similarly, replicating plasmids pREPI-UGT1A9 and pCAD1-UGDH were transformed into strain 1445 (Burke & Gould 1994 Mol Gen Genet 242, 169-176) to yield strain CAD202. Cells of all strains were aliquoted and stored at -80°C in 2x YEA medium containing 25 % glycerol until needed.

## 2. Standardisation of the Expression and Biotransformation Assay

### *2.1 Biomass production*

Cells of respective strains were transferred from permanent cultures to EMM dishes containing 5 $\mu$M thiamine and necessary supplements and were grown for 3 days at 30°C. Cell material from plates was transferred to a 10 mL EMM preculture containing the desired supplements, 20 g L-1 glucose and no thiamine in order to induce the *nmt1* promoter. From now on, all subsequent cultures were done in absence of thiamine to keep the promoter in an active state. Biomass production was scaled up by factor 10 to yield 100 mL and finally 400 mL of cell suspensions in batch cultures. Thereby, the culturing time was 1 day for all culturing stages of the parent strain NCYC2036 and 1445 while was extended to 2 days for the 10 mL and 100 mL cultures and 3 days for the 400 mL stage for the double expressors CAD202, CAD203 and CAD204.

### *2.2 Biotransformation assay*

After biomass production, the cells were centrifuged at 3000 g for 5 min and concentrated nearly 9-fold in an EMM variant containing 100 g L-1 glucose. A volume of 2 mL was used to determine the biomass dry weight of the concentrated cell suspension while a volume of 10 mL of concentrated cell suspension was then transferred to 250 mL erlenmeyer flasks and either 0.5 mL ethanol or 0.5 mL of a 10 mM ethanolic 4-methylumbelliferone (4MU) stock solution was added to the fermentation broth. The bioconversion of 4MU was carried out at 30 °C for 72 h at a shaking frequency of 150 rpm. At the end of the bioconversion assay an aliquot of 2 mL was used to determine the final biomass dry weight and the final cell suspension volume was measured.

*2.3 Sample preparation*

From each erlenmeyer flask a volume of 500 μL cell suspension was centrifuged at 1.8 × $10_4$ *g* for 10 min at the start and end of the bioconversion period to yield a clarified culture supernatant. A volume of 400 μL supernatant was then transferred to a 1.5 mL reaction tube and was mixed with 600 μL mobile phase which was subsequently centrifuged again at 1.8 x $10_4$ g for 10 min. Finally, 800 μL were transferred to a HPLC vial and stored at -4 °C until HPLC analysis.

*2.4 Analytical methods*

***2.4.1 Apparatus***

The HPLC system was as follows: a Waters integrated HPLC system consisting of Alliance 2690 separation module and a Waters 966 photodiode array detector (PDA). The system was controlled via the Millenium32 software from Waters.

***2.4.2 LC conditions***

For the simultaneous separation of 4-methylumbelliferone (4MU) and 4-methylumbelliferone glucuronide (4MUG) the LC was done in gradient mode (see Table 2). Chromatograms were extracted from the PDA channel at a wavelength of 320 nm. Standard references at 200 μM were used to gain spectra of 4MU and 4MUG and were stored in a spectra library that was used to autodetect the analytes in the PDA channel data. Integration of peaks was done on the 320 nm chromatogram by the software algorithm. Spectra extraction of detected peaks was done for all chromatograms for the time period 6 min to 15 min.

*2.5 Estimation of production rates*

Since the volume of the bioconverting cell suspension was subjected to evaporation a concentration peak area correction factor *f*con of 1.75 was determined by measuring the culture volume at the end of the fermentation period. Furthermore, the dilution factor *f*dil of 2.5 stemming from the sample preparation procedure was taken into account. A 200 μM reference of 4MUG was used to roughly estimate the peak area/concentration relation in the chromatograms yielding a reference peak area *a*ref. By noting the determined peak area a4mug of 4MUG in the chromatograms the concentration was calculated as

$$c \approx \frac{a_{4\text{mug}}}{a_{\text{ref}}} \times \frac{2.5}{1.75} \qquad (1)$$

and has the dimension "μM". By noting the length of bioconversion period of 3 days (3 d, 72 h) a production rate could be roughly computed.

*2.6 Phenotypic changes due to the expression of UGT1A9 and UGDH*

Fully induced expression in the generated, recombinant fission yeast strains (see Table 1) expressing either UGT1A9 (CAD200) or UGDH (CAD201) from the integrative vector pCAD1 yielded inconspicuous phenotypes strongly resembling the parent strain NCYC2036. However, when the second, enzymatic UGT-system component was added by transforming these strains with an autosomally replicating vector bearing the desired cDNA (Table 1) a significant growth retardation was observed under induced expression conditions. Moreover, especially strain CAD203 was additionally affected in its morphology showing appreciable deviations from the usual rod-like shape and unequal cell division. The remaining double expressors CAD204 and CAD202 were growth retarded but did not show any similar morphologic alterations.

These phenotypic changes were already a strong hint towards a functional expression. In addition, while both UGT-system components were relatively growth retarding when overex-pressed from the autosomally replicating pREP1 (Table 1) the overexpression of the human UGDH in CAD203 also affects the morphology of the host.

**3. Separation methods for UGT substrates**

It was clear from the beginning that setting up separation methods that would allow the simultaneous detection of substrate and glucuronide conjugates would be mandatory for the analysis of biotransformation assays. The determined gradient program presented in Table 2 was found to be adequate on one hand to offer a steep enough gradient to elute the usually more hydrophobic UGT substrate while on the other hand to get rid of the hydrophilic substances being present in the fermentation media. The retention times were 11.7 min $\pm$ 2 % and 8.2 min $\pm$ 2 % for 4MU and 4MUG, respectively.

**4. Biotransformation by UGT1A9 and UGDH expressing strains**

First activity assay with only 100 mL of cell suspension and with a 0.5 $\mu$M initial substrate concentration of either 4MU, 4-nitrophenol (1N), phenolphthalein (PP), 1-naphthol (1N), propofol (P), and octylgallate (OG) indicated that the 4MU bioconversion to 4MUG showed the highest detectable product formation judged from the method employed (subsection 2.4) and, therefore, was chosen as marker reaction.

Thereby, it also became clear that a significantly longer incubation time was needed in order to achieve biomass concentrations similar to the parent strains. Since concentrated biomass is able to consume the glucose present at concentrations of 20 g $L^{-1}$ within 3 h to 4 h and glucose might become depleted by the action of the heterologously expressed, human UGDH we preemptively increased the medium glucose concentration to 1.00 g $L^{-1}$.

After adapting the culturing conditions to the UGT-system expressing, recombinant fission yeast strains (see Table 1) bioconversions (see subsection 2.2) were carried out to asses the ability of the strains to bioconvert 4MU to the desired glucuronide conjugate. Figure 4 exemplarily shows a clearly detectable peak present at 8.14 min that appears only at the end of the fermentation period ($t$ = 72 h) but was not detectable at $t$ = 0 h in supernatants from a CAD203 incubation. Moreover, the parent strain NCYC2036 did not show any significant peak at that retention time neither at $t$ = 0 h nor at $t$ = 72 h (Figure 5). Furthermore, by using the stored UV/VIS spectra in the range 195 nm to 400 nm for 4MU and 4MUG it was possible to scan the peak spectra extracted from the PDA channel data for each analyzed sample. Again, while 4MU spectra were expectably detected in all incubations containing the substrate (see Figure 6 and Figure 7) the typical 4MUG spectrum gained from a pure reference standard analyzed under the same conditions only matched the mentioned peak detected at 8.14 min in the $t$ = 72 h sample from strain CAD203 (compare Figure 7b to Figure 8b). The peak spectrum match against the library is a further corroborative result demonstrating the selective and time-dependent appearance of the possible 4MU glucuronide conjugate in supernatants gained from fermentations of fission yeast strain CAD203 in presence of 4MU. This behaviour was seen in at least three independently carried out bioconversion experiments repeated at different times during a period of 4 weeks.

The same product as in case of CAD203 could also be observed in supernatants gained from fission yeast strains CAD202 and CAD204 but not in case of the parents NCYC2036 and 1445. Rate estimations (see subsection 2.5) demonstrate that the biotransforming ability of strains CAD202 and CAD204 was not as accentuated as in case of CAD203 (Table 3, not shown for strain CAD202).

Usually, at the end of the biomass growth phase the final biomass dry weight was around 2 g $L^{-1}$ for the parent strains and around 1.5 g $L^{-1}$ for the double expressors with cells being in the stationary growth phase. The concentration procedure (see subsection 2.2) yielded biomass dry weight concentrations around 18 g $L^{-1}$ for the parent strains and around 14 g $L^{-1}$ for the double expressors CAD202, CAD203 and CAD204. However, during the fermentation process in presence of 100 g $L^{-1}$ glucose fission yeast strain CAD203 increased its biomass with a nearly doubled rate compared to NCYC2036 and CAD204. At the end of the fermentation all glucose was consumed and the pH dropped from 5.5 to values between 2.8 and 3.4 depending on strain and substrate.

**Table 1:**

**Fission yeast strains constructed according to Example 1.**

Table 1: Fission yeast strains

| strain | genotype | parent | protein | replication | # exp.[1] |
|---|---|---|---|---|---|
| NCYC2036 | *$h^-$ uraf dlis* | - | none | - | none |
| CAD200 | *$h^-$ tras.diss::pCADI-UGTIA9* | NCYC2036 | UGT1A9 | chromosomal | 1 |
| CAD201 | *$h^-$ tras.diss::pCAD1-UCDH* | NCYC2036 | UGDH | chromosomal | 1 |

(continued)

| strain | genotype | parent | protein | replication | # exp.[1] |
|---|---|---|---|---|---|
| CAD203 | *h⁻ tras.diss::pCAD1-UGT1A9/pREP1-UGDH* | CAD200 | UGT1A9<br>UGDH | chromosomal<br>autosomal | 1<br>many |
| CAD204 | *h⁻ tras.diss::pCAD1-UGDH/pREP1-UGT1A9* | CAD201 | UGDH<br>UGT1A9 | chromosomal<br>autosomal | 1<br>many |
| 1445 | *h⁻ ade6.M210 leu1.32 urf. diis his3Δ1* | - | none | - | none |
| CAD202 | *h⁻ ade3M210 leu1.32 urf. diis his9.Δ1 /pGAD1-UGDH/ pREP1-UGT1A9* | 1445 | UGDH<br>UGT1A9 | autosomal<br>autosomal | many<br>many |

1: number of expression cassettes per cell

**Table 2:**
**HPLC gradient elution for the simultaneous detection of 4MU and 4MUG**

| time range | AcN [%] | 0.1 % HAc [%] | transition |
|---|---|---|---|
| 0 min to 5 min | 5 | 95 | no |
| 5 min to 10 min | 30 | 70 | linear |
| 10 min to 12 min | 30 | 70 | no |
| 12 min | 5 | 95 | step |
| 12 min to 15 min | 5 | 95 | no |

**Table 3:**
**Averaged parameters observed during bioconversion of 4MU**

| strain | c4mug1 [M] | rate2 [mol L-1 d-1] | cdw3 [gL-1] |
|---|---|---|---|
| NCYC2036 | - | - | 9 to 11 |
| CAD203 | 140 to 200 | 47 to 67 | 14 to 18 |
| CAD204 | 30 to 50 | 10 to 17 | 8 to 10 |

1: 4-methylumbelliferone glucuronide concentration at end of fermentation
2: estimated 4-methylumbelliferone glucuronide production rate
3: biomass dry weight increase during the fermentation period

**Claims**

1. Expression system for a functional UDP-glucuronosyl-transferase (UTG) comprising at least one yeast cell population of recombinant *Schizosaccharomyces pombe,* which is transformed with the nucleic acid sequence encoding UTG and/or the nucleic acid sequence encoding UDP-glucose dehydrogenase (UGDH).

2. Expression system according to claim 1, wherein the nucleic acid sequence encoding UDP-glucuronosyl-transferase derives from the sequence encoding the UTG, which is selected from the group consisting of rat, mouse, pig, dog, sheep, goat, cow, horse, monkey, mammal including human UTGs, their variants and functional variants.

3. Expression system according to claims 1 or 2, wherein the nucleic acid sequence encoding UDP-glucose dehydrogenase derives from the sequence encoding the UTG, which is selected from the group consisting of rat, mouse, pig, dog, sheep, goat, cow, horse, monkey, mammal including human UGDHs, their variants and functional variants.

4. Expression system according to anyone of the previous claims, wherein the nucleic acid sequence encoding UTG and/or the nucleic acid sequence encoding UGDH is transferred to the yeast cells by an integrative or autosomally replicating gene transfer vector.

5. Expression system according to anyone of the previous claims, wherein the nucleic acid sequence encoding UTG and/or the nucleic acid sequence encoding UGDH is under the control of at least one regulatory element, which is selected from the group consisting of inducible promoters, homologous household promoters and heterologous promoters.

6. Expression system according to anyone of the previous claims, wherein the ration of the expression of functional UTG:UGDH is between 1:1 and 1:2.5.

7. Use of the expression system according to anyone of the previous claims comprising the recombinant *Schizosaccharomyces pombe,* which is transformed with the nucleic acid sequence encoding UTG and the nucleic acid sequence encoding UGDH for the production of glucuronides.

8. Method for the detection and/or synthesis of glucuronides comprising the steps of

   - culturing under suitable conditions the expression system according to anyone of the previous claims 1 to 6 comprising at least one cell population of recombinant *Schizosaccharomyces pombe,* which is transformed with the nucleic acid sequence encoding UTG and the nucleic acid sequence encoding UGDH,
   - adding a chemical substance, drug, xenobiotic or endogenous substance to the fermentation broth of the cell culture,
   - collecting the supernatant,
   - optionally, analysing the supernatant by HPLC or MS, and
   - extracting the desired glucuronide from the supernatant.

9. Use of the expression system according to anyone of the previous claims 1 to 6 comprising the recombinant *Schizosaccharomyces pombe,* which is transformed the nucleic acid sequence encoding UDP-glucose dehydrogenase (UGDH) for the production or UDP-glucuronic acid (UDP-GA).

10. Method for synthesis of UDP-glucuronic acid (UDP-GA) comprising the steps of

- culturing under suitable conditions the expression system according to any of the claims 1 to 6 comprising at least one cell population of recombinant *Schizosaccharomyces pombe,* which is transformed with the nucleic acid sequence encoding UDP-glucose dehydrogenase (UGDH),
- collecting the supernatant,
- optionally, analysing the supernatant by HPLC, and
- extracting the desired UDP-glucuronic acid (UDP-GA) from the supernatant.

**11.** Use of the expression system according to any of the claims 1 to 6 comprising the recombinant *Schizosaccharomyces pombe,* which is transformed with the nucleic acid sequence encoding UTG for the identification of UTG modulators.

**12.** Method for the identification of UTG modulators comprising the steps of

- culturing under suitable conditions the expression system according to claim 1 comprising at least one cell population of recombinant *Schizosaccharomyces pombe,* which is transformed with the nucleic acid sequence encoding UDP-glucuronosyltransferase (UTG) and the nucleic acid sequence encoding UDP-glucose dehydrogenase (UGDH),
- establishing the bioconversion assay with a reference substance,
- adding a test substance to the fermentation broth,
- analysing the supernatant for the presents of UTG dependent metabolites.

**13.** Use of the expression system according to anyone of the previous claims 1 to 6 comprising the recombinant *Schizosaccharomyces pombe,* which is transformed with the nucleic acid sequence encoding UTG and the nucleic acid sequence encoding UGDH for the manufacturing of an enzyme preparation

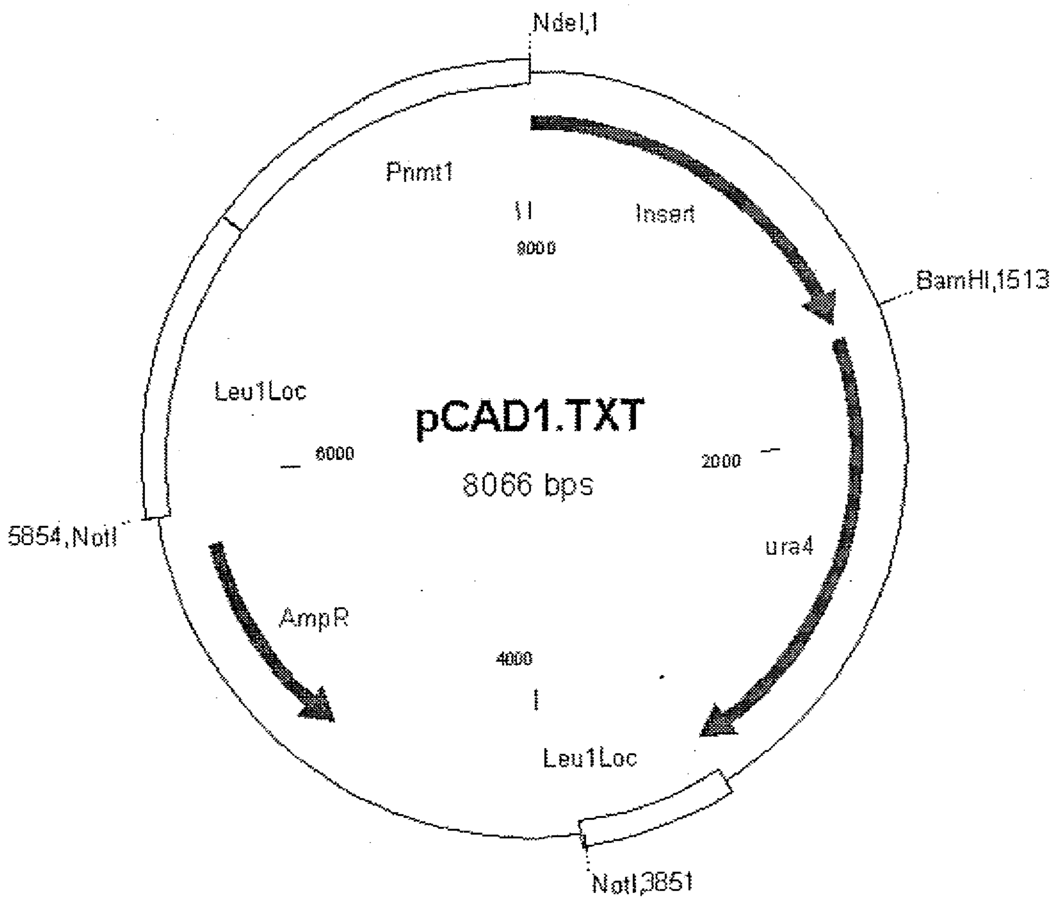
NdeI,1
Pnmt1
Insert
8000
BamHI,1513
Leu1Loc
pCAD1.TXT
8066 bps
6000
2000
5854,NotI
ura4
AmpR
4000
Leu1Loc
NotI,3851

Fig 1

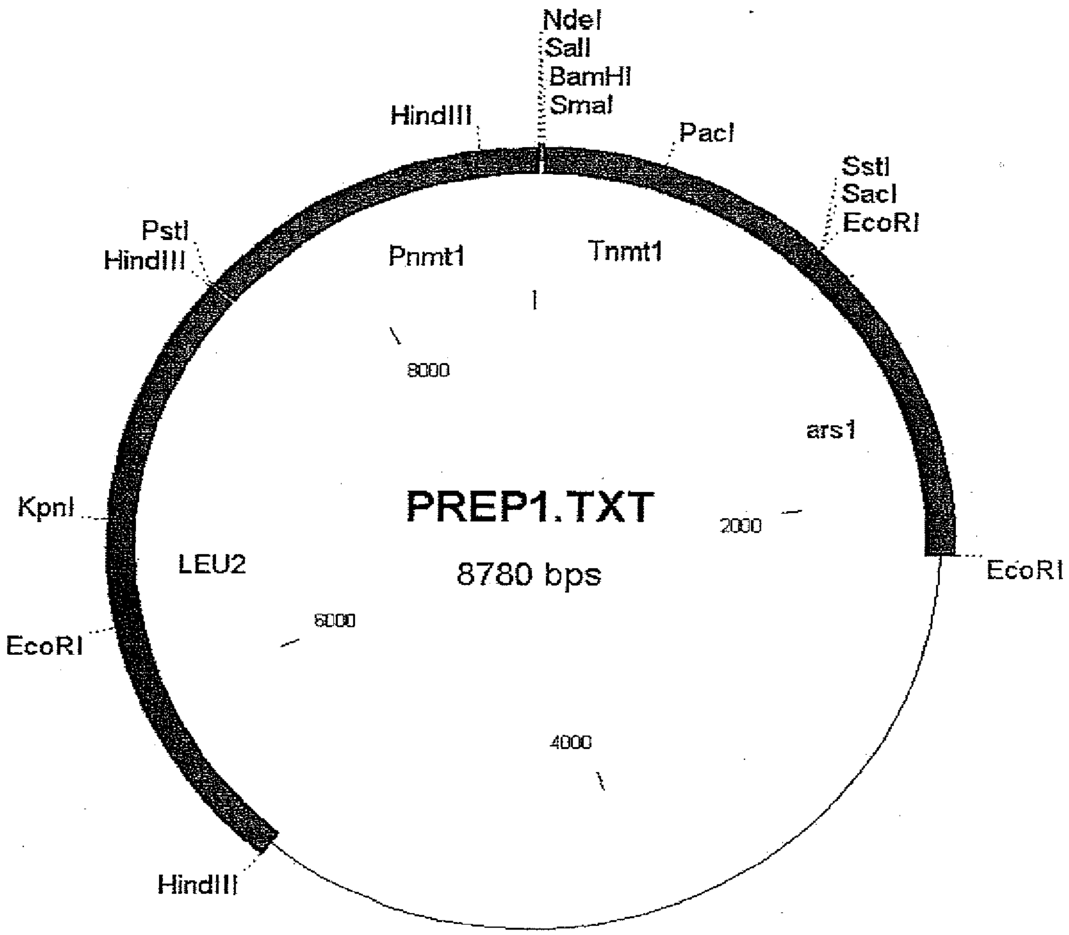
NdeI
SalI
BamHI
SmaI
HindIII
PacI
SstI
SacI
EcoRI
PstI
HindIII
Pnmt1
Tnmt1
1
8000
ars1
KpnI
PREP1.TXT
8780 bps
2000
EcoRI
LEU2
6000
EcoRI
4000
HindIII

Fig 2

5'-**CATAT G**ATGG CTTGC ACAGG GTGGA CCAGC CCCCT TCCTC TATGT
GTGTG TCTGC TGCTG ACCTG TGGCT TTGCC GAGGC AGGGA AGCTA CTGGT
AGTGC CCATG GATGG GAGCC ACTGG TTCAC CATGA GGTCG GTGGT GGAGA
AACTC ATTCT CAGGG GGCAT GAGGT GGTTG TAGTC ATGCC AGAGG TGAGT
TGGCA ACTGG GAAGA TCACT GAATT GCACA GTGAA GACTT ATTCA ACTTC
ATATA CCCTG GACCA TCTGG ACCCG GAGTT CAAGG CTTTT GCCCA TGCTC
AATGG AAAGC ACAAG TACGA AGTAT ATATT CTCTA TTAAT GGGTT CATAC
AATGA CATTT TTGAC TTATT TTTTT CAAAT TGCAG GAGTT TGTTT AAAGA
CAAAA AATTA GTAGA ATACT TAAAG GAGAG TTCTT TTGAT GCAGT GTTTC
TCGAT CCTTT TGATA ACTGT GGCTT AATTG TTGCC AAATA TTTCT CCCTC
CCCTC CGTGG TCTTC GCCAG GGGAA TACTT TGCCA CTATC TTGAA GAAGG
TGCAC AGTGC CCTGC TCCTC TTTCC TATGT CCCCA GAATT CTCTT AGGGT
TCTCA GATGC CATGA CTTTC AAGGA GAGAG TACGG AACCA CATCA TCCAC
TTGGA GGAAC ATTTA TTATG CCACC GTTTT TTCAA AAATG CCCTA GAAAT
AGCCT CTGAA ATTCT CCAAA CACCT GTTAC GGAGT ATGAT CTCTA CAGCC
ACACA TCAAT TTGGT TGTTG CGAAC GGACT TTGTT TTGGA CTATC CCAAA
CCCGT GATGC CCAAC ATGAT CTTCA TTGGT GGTAT CAACT GCCAT CAGGG
AAAGC CGTTG CCTAT GGAAT TTGAA GCCTA CATTA ATGCT TCTGG AGAAC
ATGGA ATTGT GGTTT TCTCT TTGGG ATCAA TGGTC TCAGA AATTC CAGAG
AAGAA AGCTA TGGCA ATTGC TGATG CTTTG GGCAA AATCC CTCAG ACAGT
CCTGT GGCGG TACAC TGGAA CCCGA CCATC GAATC TTGCG AACAA CACGA
TACTT GTTAA GTGGC TACCC CAAAA CGATC TGCTT GGTCA CCCGA TGACC
CGTGC CTTTA TCACC CATGC TGGTT CCCAT GGTGT TTATG AAAGC ATATG
CAATG GCGTT CCCAT GGTGA TGATG CCCTT GTTTG GTGAT CACAT CCACA
ATGCA AAGCG CATGG AGACT AAGGG AGCTG GAGTG ACCCT GAATG TTCTG
GAAAT GACTT CTGAA GATTT AGAAA ATGCT CTAAA AGCAG TCATC AATGA
CAAAA GTTAC AAGGA GAACA TCATG CGCCT CTCCA GCCTT CACAA GGACC
GCCCG GTGGA GCCGC TGGAC CTGGC CGTGT TCTGG GTGGA GTTTG TGATG
AGGCA CAAGG GCGCG CCACA CCTGC GCCCC GCAGC CCACG ACCTC ACCTG
GTACC AGTAC CATTC CTTGG ACGTG ATTGG TTTCC TCTTG GCCGT CGTGC
TGACA GTGGC CTTCA TCACC TTTAA ATGTT GTGCT TATGG CTACC GGAAA
TGCTT GGGGA AAAAA GGGCG AGTTA AGAAA GCCCA CAAAT CCAAG ACCCA
TTGA**G GATCC**-3'

Fig. 3

5′-**CATAT G**ATGT TTGAA ATTAA GAAGA TCTGT TGCAT CGGTG CAGGC TATGT
TGGAG GACCC ACATG TAGTG TCATT GCTCA TATGT GTCCT GAAAT CAGGG
TAACG GTTGT TGATG TCAAT GAATC AAGAA TCAAT GCGTG GAATT CTCCT
ACACT TCCTA TTTAT GAGCC AGGAC TAAAA GAAGT GGTAG AATCC TGTCG
AGGAA AAAAT CTTTT TTTTT CTACC AATAT TGATG ATGCC ATCAA AGAAG
CTGAT CTTGT ATTTA TTTCT GTGAA TACTC CAACA AAAAC CTATG GAATG
GGGAA AGGCC GGGCA GCAGA TCTGA AGTAT ATTGA AGCTT GTGCT AGACG
CATTG TGCAA AACTC AAATG GGTAC AAAAT TGTGA CTGAG AAAAG CACAG
TTCCA GTGCG GGCAG CAGAA AGTAT CCGTC GCATA TTTGA TGCAA ACACA
AAACC CAACT TGAAT TTACA GGTGC TGTCC AACCC TGAGT TTCTG GCAGA
GGGAA CAGCC ATCAA GGACC TAAAG AACCC AGACA GAGTA CTGAT TGGAG
GGGAT GAAAC TCCAG AGGGC CAGAG AGCTG TGCAG GCCCT GTGTG CTGTA
TATGA GCACT GGGTT CCCAG AGAAA AGATC CTCAC CACTA ATACT TGGTC
TTCAG AGCTT TCCAA ACTGG CAGCA AATGC TTTTC TTGCC CAGAG AATAA
GCAGC ATTAA CTCCA TAAGT GCTCT GTGTG AAGCA ACAGG AGCTG ATGTA
GAAGA GGTAG CAACA GCGAT TGGAA TGGAC CAGAG AATTG GAAAC AAGTT
TCTAA AAGCC AGTGT TGGGT TTGGT GGGAG CTGTT TCCAA AAGGA TGTTC
TGAAT TTGGT TTATC TCTGT GAGGC TCTGA ATTTG CCAGA AGTAG CTCGT
TATTG GCAGC AGGTC ATAGA CATGA ATGAC TACCA GAGGA GGAGG TTTGC
TTCCC GGATC ATAGA TAGTC TGTTT AATAC AGTAA CTGAT AAGAA GATAG
CTATT TTGGG ATTTG CATTC AAAAA GGACA CTGGT GATAC AAGAG AATCT
TCTAG TATAT ATATT AGCAA ATATT TGATG GATGA AGGTG CACAT CTACA
TATAT ATGAT CCAAA AGTAC CTAGG GAACA AATAG TTGTG GATCT TTCTC
ATCCA GGTGT TTCAG AGGAT GACCA AGTGT CCCGG CTCGT GACCA TTTCC
AAGGA TCCAT ATGAA GCATG TGATG GTGCC CATGC TGTTG TTATT TGCAC
TGAGT GGGAC ATGTT TAAGG AATTG GATTA TGAAC GCATT CATAA AAAAA
TGCTA AAGCC AGCCT TTATC TTCGA TGGAC GGCGT GTCCT GGATG GGCTC
CACAA TGAAC TACAA ACCAT TGGCT TCCAG ATTGA AACAA TTGGC AAAAA
GGTGT CTTCA AAGAG AATTC CATAT GCTCC TTCTG GTGAA ATTCC GAAGT
TTAGT CTTCA AGATC CACCT AACAA GAAAC CTAAA GTGTA G**GGAT CC**-3′

Fig. 4

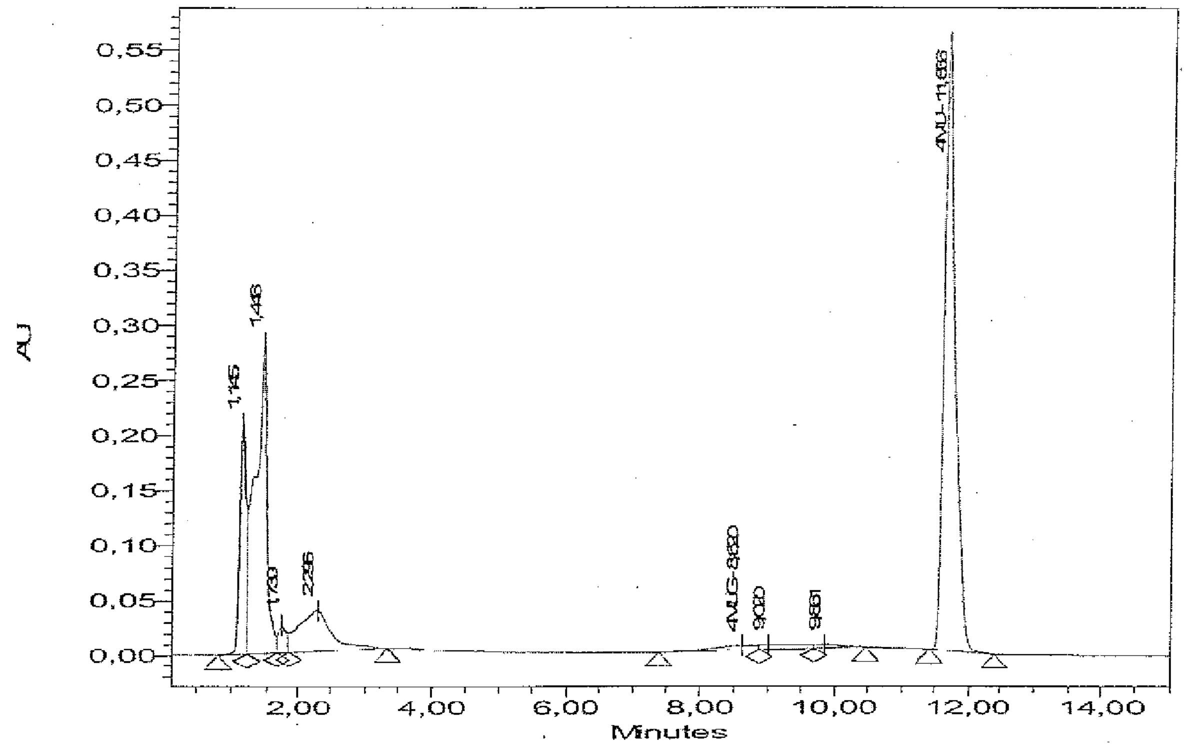


(a) $t = 0$ h

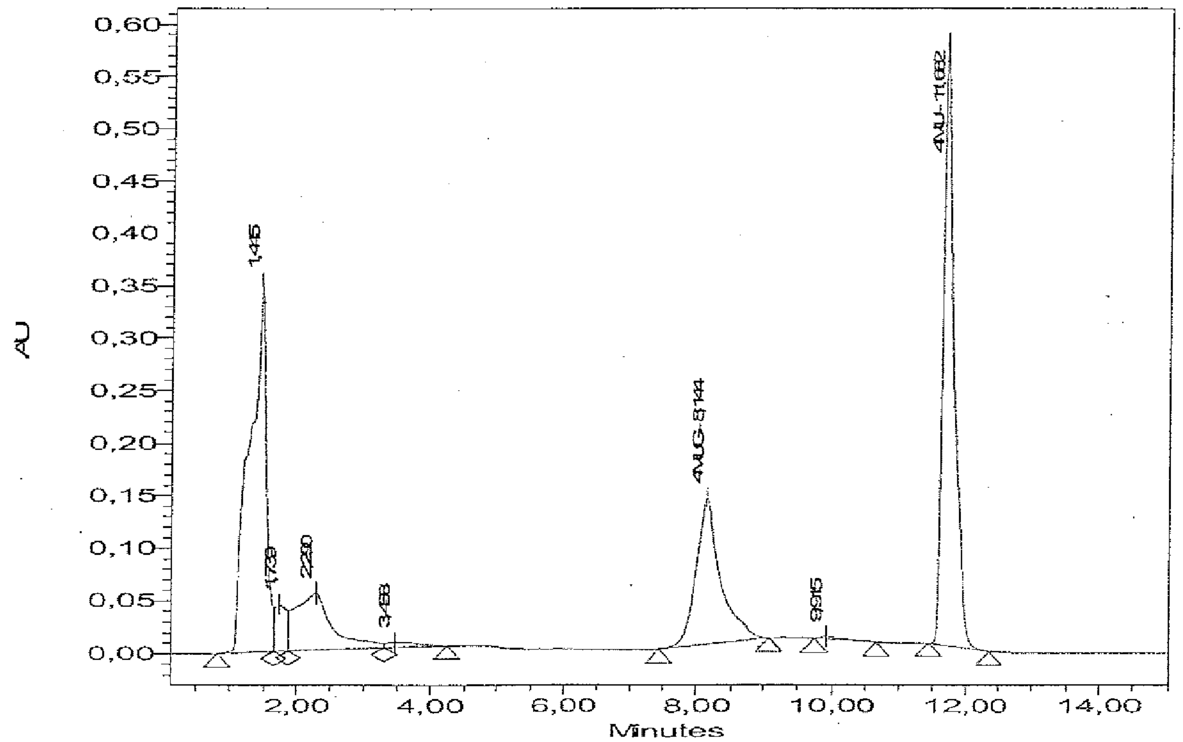


(b) $t = 72$ h

Fig.: 5

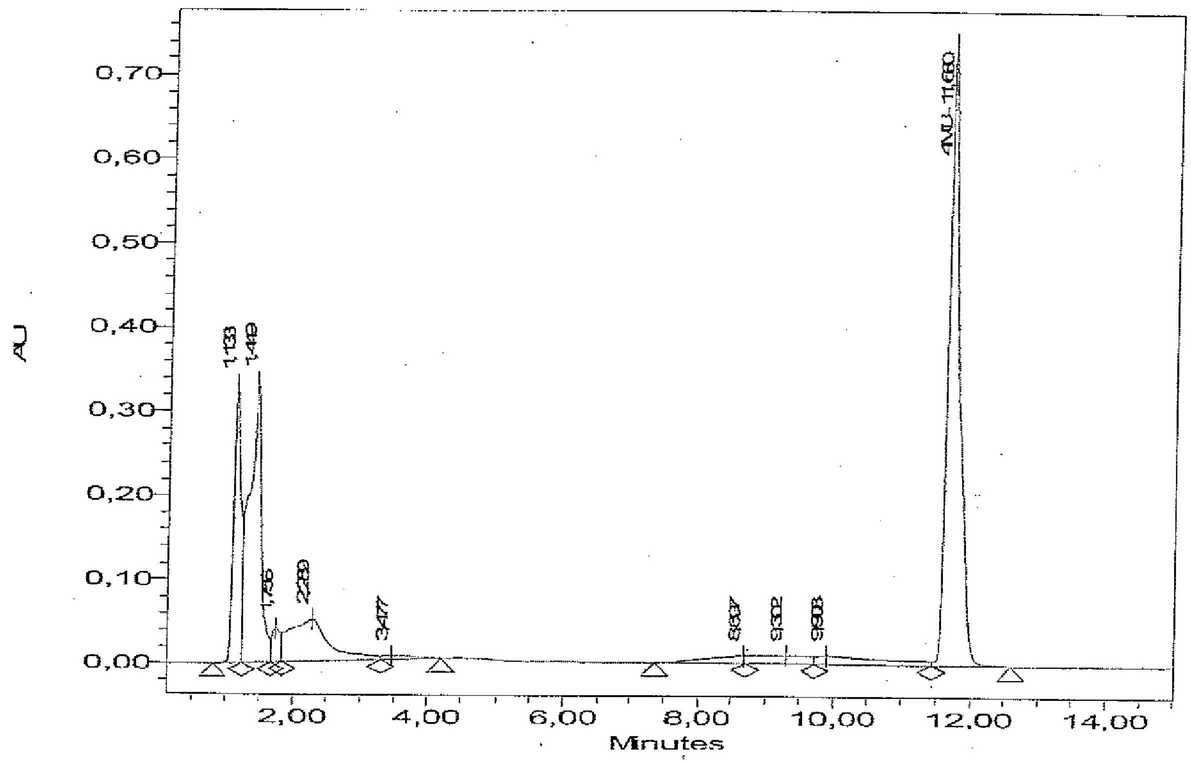


(b) $t = 72$ h

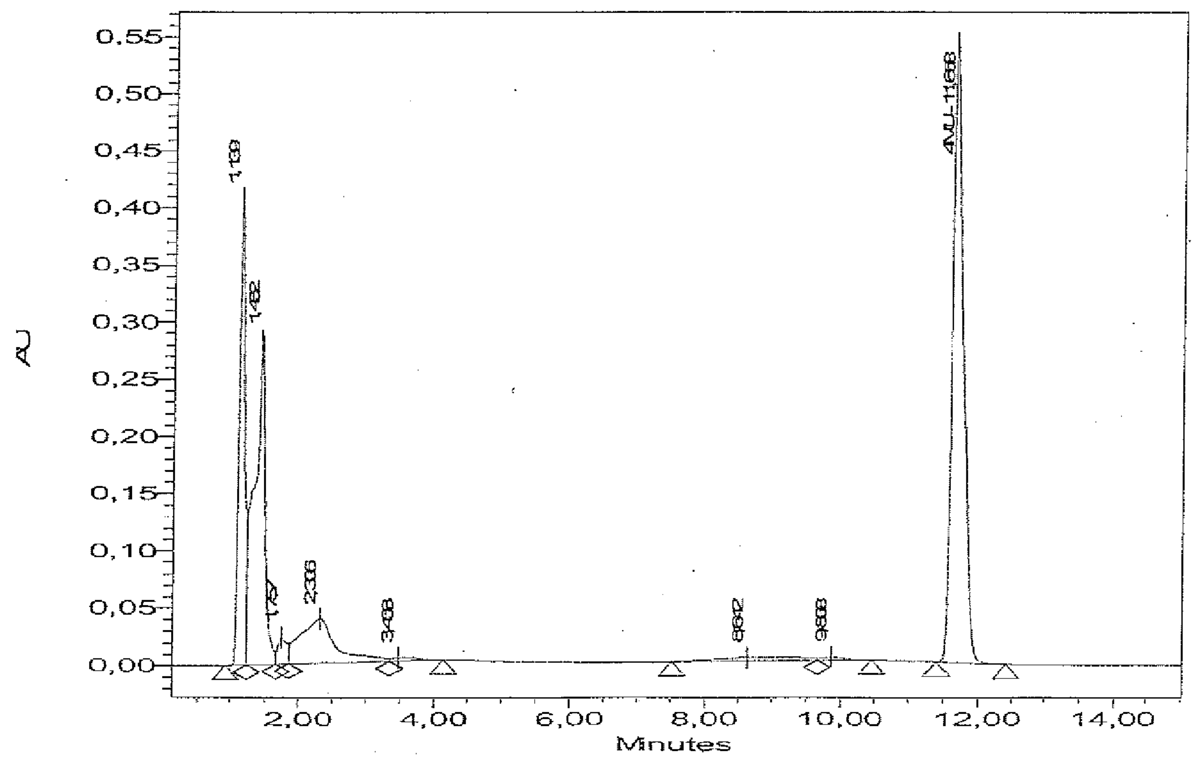


(a) $t = 0$ h

Fig.: 6

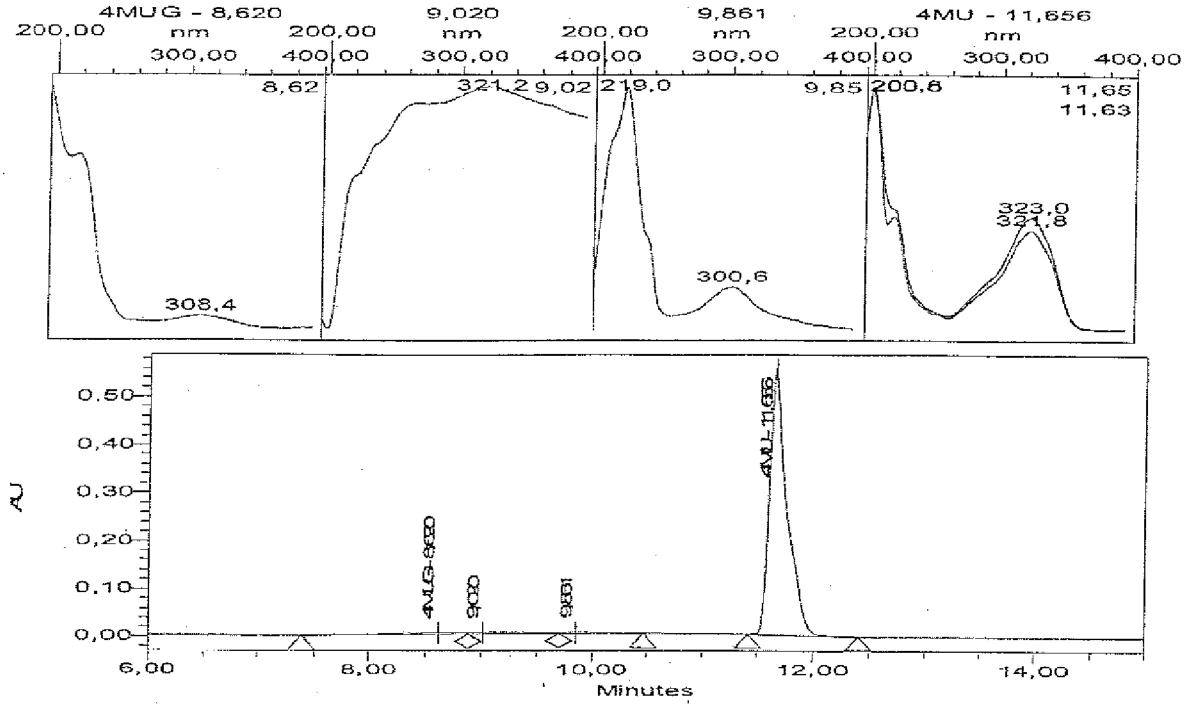


(a) $t = 0$ h

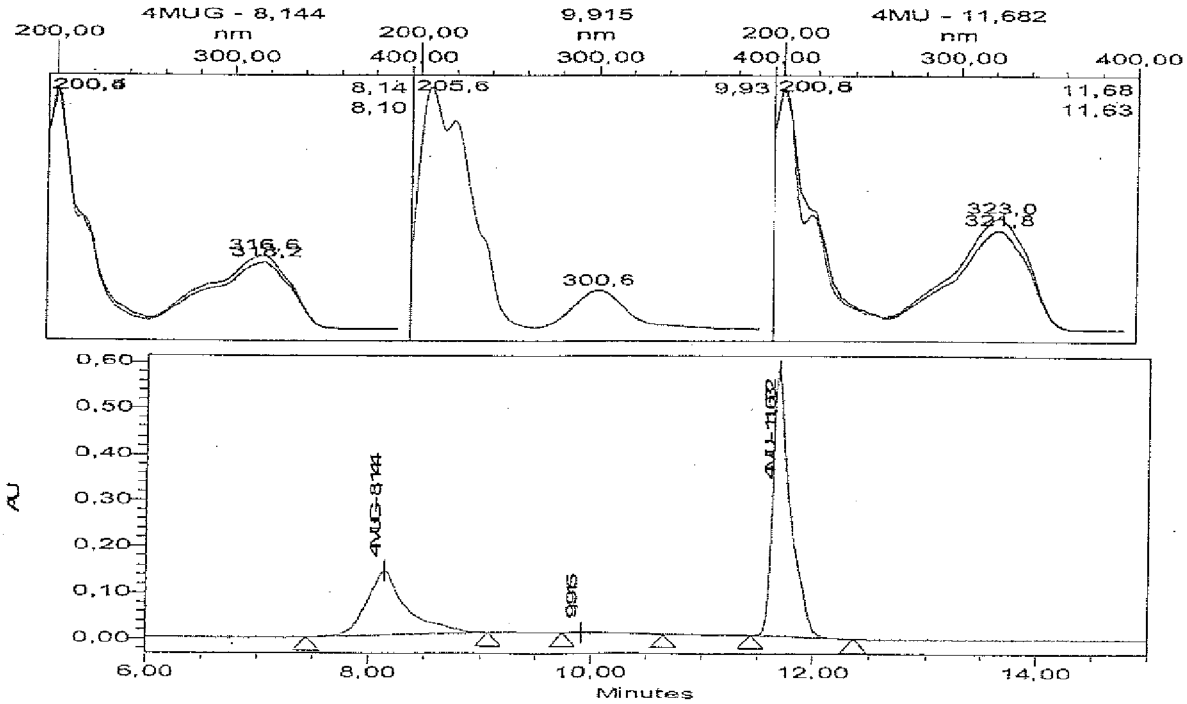


(b) $t = 72$ h

Fig.: 7

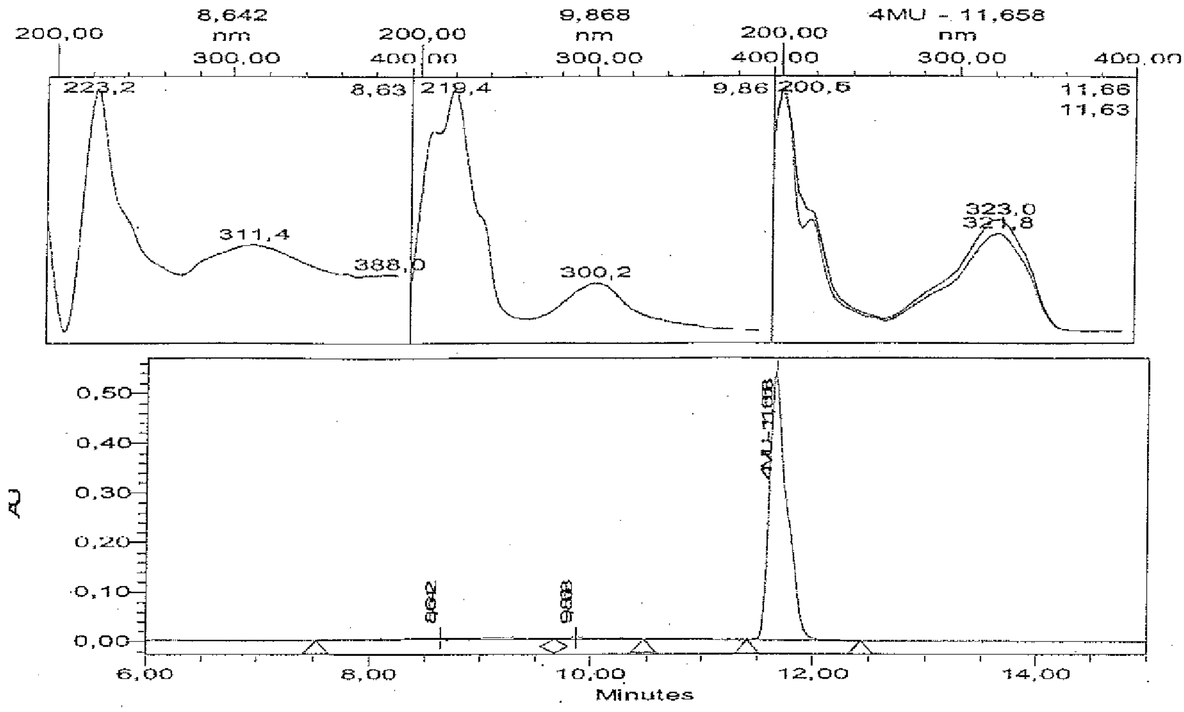


(a) $t = 0$ h

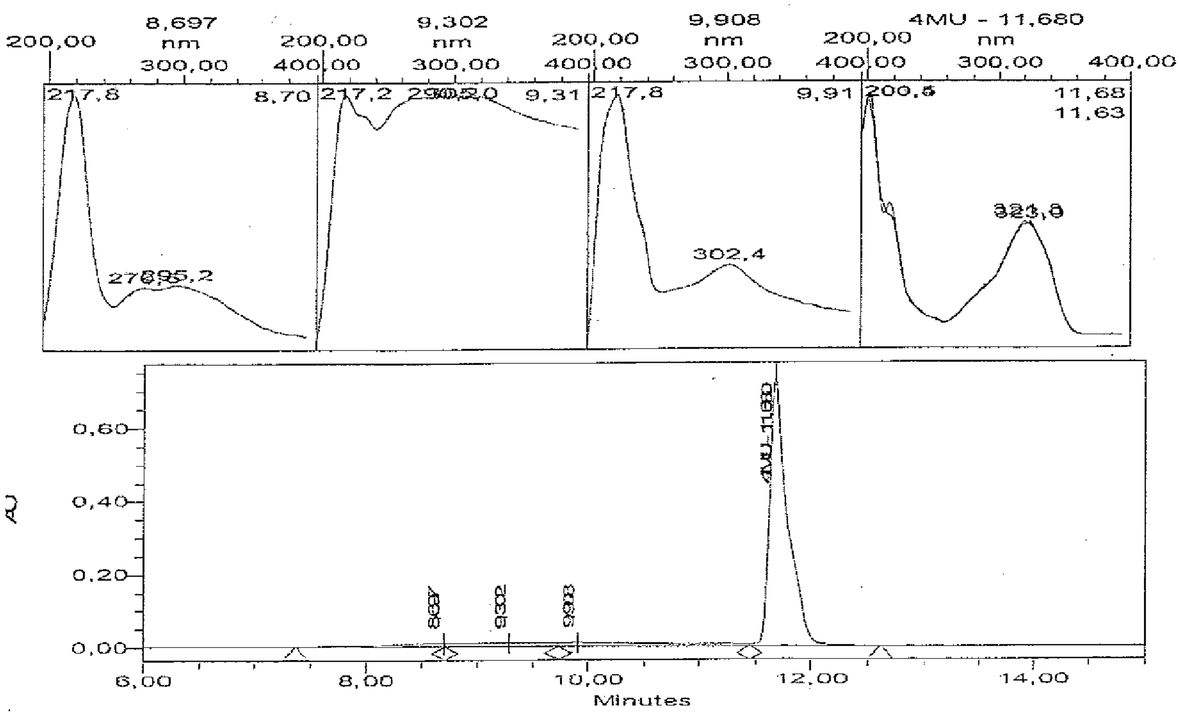


(b) $t = 72$ h

Fig. 8

Europäisches Patentamt
European Patent Office
Office européen des brevets

# EUROPEAN SEARCH REPORT

Application Number
EP 08 16 4826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOGHROL F ET AL: "Expression of UDP-glucuronosyltransferase cDNA in Saccharomyces cerevisiae as a membrane-bound and as a cytosolic form." BIOCHEMISTRY 6 MAR 1990, vol. 29, no. 9, 6 March 1990 (1990-03-06), pages 2349-2356, XP002513380 ISSN: 0006-2960 * the whole document * ----- | 1-5 | INV. C12N9/04 C12N9/10 C12N15/81 |
| X | DATABASE WPI Week 200759 Thomson Scientific, London, GB; AN 2007-617536 XP002513381 -& JP 2007 174957 A (TOYOBO KK) 12 July 2007 (2007-07-12) * abstract; figure 2 * ----- | 1-5,9,10 | |
| D,A | RADOMINSKA-PANDYA ANNA ET AL: "A historical overview of the heterologous expression of mammalian UDP-glucuronosyltransferase isoforms over the past twenty years." CURRENT DRUG METABOLISM APR 2005, vol. 6, no. 2, April 2005 (2005-04), pages 141-160, XP008101457 ISSN: 1389-2002 * the whole document * ----- | 1-13 | TECHNICAL FIELDS SEARCHED (IPC) C07K C12N |

The present search report has been drawn up for all claims

| Place of search | Date of completion of the search | Examiner |
|---|---|---|
| The Hague | 4 February 2009 | Spindler, Mark-Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 08 16 4826

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2009

| Patent document cited in search report | Publication date | Patent family member(s) | Publication date |
|---|---|---|---|
| JP 2007174957 A | 12-07-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Radominska-Pandya et al.** *Current Drug Metabolism,* 2005, vol. 6, 141-160
- **Ikushiro et al.** *Biochimica et Biophysica Acta,* 2004, vol. 1672, 86-92
- **Oeda et al.** *DNA,* 1985, vol. 4, 203-210
- **Wood et al.** *Nature,* 2002, vol. 415, 871-880
- **Trubetskoy et al.** *J Pharm Pharmacol,* 2008, vol. 60, 1061-1067
- **Meech ; Mackenzie.** *Arch Biochem Biophys.,* 1998, vol. 356, 77-85
- **Radominska-Pandya et al.** *Arch Biochem Biophys.,* 2002, vol. 399, 37-48
- **Radominska-Pandya et al.** *Curr Drug Metab.,* 2005, vol. 6, 141-160
- **Mackenzie et al.** *Pharmacogenet Genomics.,* 2005, vol. 15, 677-685
- **Huh et al.** *J Biol Chem.,* 2004, vol. 279, 37491-37498
- **Sambrook et al.** Molecular Cloning: A Laboratory Manual. Cold Spring Harbor Press, 1989
- **Moreno et al.** *Methods Enzymol,* 1991, vol. 194, 795-823
- **Alfa et al.** Experiments with fission yeast. A laboratory course manual. Cold Spring Harbor Press, 1993
- **Dragan et al.** *FEMS Yeast Res,* 2005, vol. 5, 621-625
- **Losson ; Lacroute.** *Cell,* 1983, vol. 32, 371-377
- **Burke ; Gould.** *Mol Gen Genet,* 1994, vol. 242, 169-176